(19) **Europäisches Patentamt / European Patent Office / Office européen des brevets**

(11) **EP 1 941 859 A1**

(12) **EUROPEAN PATENT APPLICATION**

(43) Date of publication:
09.07.2008 Bulletin 2008/28

(51) Int Cl.:
*A61K 8/06* (2006.01)     *A61K 8/25* (2006.01)
*A61K 8/27* (2006.01)     *A61K 8/29* (2006.01)
*A61K 8/365* (2006.01)    *A61K 8/81* (2006.01)
*A61Q 19/00* (2006.01)

(21) Application number: 06126376.0

(22) Date of filing: 18.12.2006

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK RS

(71) Applicant: ACO Hud Nordic AB
194 26 Upplands Väsby (SE)

(72) Inventors:
• Sahlin, Anna
191 49, Sollentuna (SE)

• Lodén, Marie
170 77, Solna (SE)
• Åkerström, Ulf
117 62, Stockholm (SE)
• Neimert, Kristina
192 53, Sollentuna (SE)

(74) Representative: Holmberg, Nils Anders Patrik et al
BRANN AB
Västgötagatan 2
P.O. Box 17192
104 62 Stockholm (SE)

(54) **Topical formulations**

(57) The present invention relates to formulations based on a w/o emulsion for topical application containing a stinging agent and microparticles effective for follicular closure, so that the composition has a reduced stinging potential and an improved feel. The formulations are intended for use as a cosmetic or pharmaceutical formulation. The invention also relates to the use of such w/o emulsions for preparing cosmetic and pharmaceutical topical formulations, and the use of microparticles in a formulation for topical application comprising a w/o emulsion.

Figure 3
Degree of perceived stinging with time from Formulation 1 and Formulation 7.

**Description**

**Technical field of the invention**

The present invention generally relates to formulations based on a w/o emulsion for topical application containing a stinging agent, and microparticles effective for follicular closure. The formulations are intended for use as a cosmetic or pharmaceutical formulation. The invention also relates to the use of such w/o emulsions for preparing cosmetic and pharmaceutical topical formulations having reduced stinging potential, as well as the use of microparticles in a formulation for topical application comprising a w/o emulsion for reducing the stinging potential of a stinging agent included in the formulation

**Background art**

Epidemiological surveys indicate that 50% or more of female consumers believe they have sensitive skin. Up to 12% of the general population also report adverse skin reactions to personal care products. Subjective sensations (no signs of inflammation), such as smarting, burning and stinging immediately after application of a formulation may be one of the most commonly experienced adverse reactions.

Substances known to cause subjective sensations include *i.a.* lactic acid, which may for example be included as a humectant in a topical formulation. Lactic acid is also known for its beneficial dermatological effects. However, in the art ingredients that can cause adverse reactions tend to be avoided as far as possible.

WO0113876 generally relates to skin cosmetic compositions containing a weak carboxylic acid. WO0113876 discloses that hydroxy acids and other weak carboxylic acids when used at high concentrations occasionally may be associated with skin irritation, such as redness and stinging sensation upon application. Water soluble weak acids when delivered from an o/w emulsion at acidic pH often induce high levels of sting. The irritation can be ameliorated by lowering the amount of active ingredient in the composition or by reducing the active's penetration through the skin. A serious drawback of both approaches is that the efficacy of the active is impaired. The weak acid related irritation can be reduced by raising the composition's pH but this method yields reduced efficacy due to a reduced acid penetration through the skin. To overcome the problem, WO0113876 instead uses in an o/w skin cosmetic composition a specified amount of a random copolymer of ethylene oxide and propylene oxide of a specified molecular weight.

US 6,440,432 offers an alternative solution to the problem of WO0113876, according to which a certain amount of a dextran or maltodextrin is used in an o/w cosmetic skin composition containing a weak carboxylic acid to overcome the problem.

WO0113877 offers yet an alternative solution to the problem of WO0113876, according to which polyethylene glycol is used in an o/w cosmetic skin composition containing a weak carboxylic acid to overcome the problem.

Sah et al. in the article entitled "An in vitro study of the effects formulation variables and product structure on percutaneous absorption of lactic acid", J. Cosmet. Sci., 49, 257-273 (1998), compares w/o, o/w and w/o/w emulsions of the formula of paraffin oil 35% w/w, Hypermer A60 2.8% w/w, Synperonic (a block copolymer of ethylene oxide and propylene oxide) 1.2% w/w, lactic acid 8% w/w, pH adjuster, KOH, and balance water. According to the authors, the rapid hydration (and consequent water loss from the skin) that occurs when an o/w emulsion film is applied to the skin often leads to a very high peak ("bolus effect") in the active flux, which, at low pH, can cause consumer-perceived negatives such as sting. On comparing the different emulsions it was found that the w/o and w/o/w emulsions delivered less lactic acid to the skin, and produced a lower skin hydration than the o/w emulsion, leading the authors to suggest that the w/o and w/o/w emulsions should lead to a more controlled skin uptake of lactic acid. Sah et al. also notes that only a few reports of systematic study on uptake of AHAs to various skin strata from topical application, and moreover that the results of some of them appear to be in conflict.

It is an object of the present invention to provide a topical formulation having improved sting reducing potential and skin feel.

For a formulation for topical application of the preamble of claim 1, comprising a water-in-oil emulsion of an emollient-containing oily phase and an aqueous phase, a stinging agent, and an emulsifier, the problem has been solved by means of including in the formulation microparticles of a size effective for follicular closure.

**Summary of invention**

The present inventors have found that lactic acid produced less stinging when formulated in a w/o emulsion, as compared to a conventional o/w emulsion.

The present inventors have further found skin appendages, such as follicles, to be a transport pathway for lactic acid, delivered to the skin from an emulsion by topical application thereto, which is associated with stinging.

The present inventors have found that follicular transport can be reduced by including in the formulation microparticles effective for follicular closure.

The microparticles will also impart a powdery and dry feel to the formulation, and thus improved cosmetic properties. The tackiness associated with the inclusion of mineral oils, such as paraffin oil, will thus be counteracted or reduced by the particles.

By using the emulsifiers of the inventive formulations the oil content of the formulation can be reduced to the level of a conventional o/w emulsion, such as e.g. 10-25%, thereby further improving the cosmetic properties of the formulation, and reducing the generally perceived oily feel of a w/o emulsion. The combination of the emulsifiers and the hydrophilic emollients is also believed to have a synergistic anti-stinging effect, due to their high spreading abilities which facilitate a uniform coverage of the surface irregularities and orifices.

Accordingly, the topical formulation of the present invention allows for inclusion of stinging ingredients, which ingredients may be desirable to include in the formulation for their beneficial properties as humectants, conservatives, and/or as pharmaceutically active substances. Obviously, any pharmaceutically active substances contained in the formulation of the invention are not restricted to stinging substances, but can be any desirable pharmaceutically active substance for topical administration.

Also, by virtue of reduced stinging and improved cosmetic properties the inventive formulation is believed to enhance the tolerability of the formulations used for treating atopics and other patients with eczema or hyperkeratotic skin.

Consequently, in one aspect the present invention relates to a formulation for topical application. In one embodiment the formulation is intended for use as a cosmetic. In another embodiment the formulation is intended for use as pharmaceutical.

In a further aspect the present invention relates to the use of a w/o emulsion, containing microparticles effective for follicular closure, for preparing a topical formulation containing a stinging ingredient having reduced stinging potential.

In yet an aspect the invention relates to the use of microparticles effective for follicular closure in a formulation for topical application comprising a w/o emulsion for reducing the stinging potential of a stinging agent included in the formulation.

Further objects, embodiments and advantages of the present invention will be apparent from the detailed description and appended claims.

**Brief description of the attached drawings**

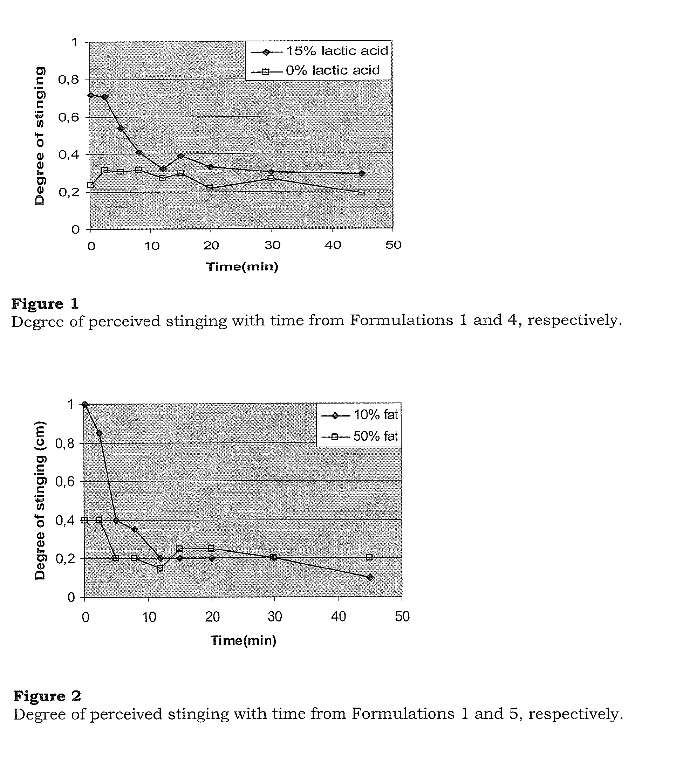

Figure 1 shows the degree of perceived stinging with time from the comparative formulation 1 with 15% lactic acid and comparative formulation 4 without lactic acid with pH adjusted to 3 using hydrochloric acid. The degree of stinging was marked on a 9 cm visual analogue scale. Median values, N=20. Significant differences between maximum degree of stinging (p=0.003) and area under curve (AUC) (p=0.022), demonstrates the stinging capacity of lactic acid.

Figure 2 shows the degree of perceived stinging with time from formulation 1 having 15 % lactic acid in the o/w-emulsion with 10% fat and from formulation 5 with 50% fat. The degree of stinging was marked on a 9 cm visual analogue scale. Median values, N=19. The maximum degree of stinging tended to be lower from the 50% fat formulation than from the 10% fat formulation (p=0.077).

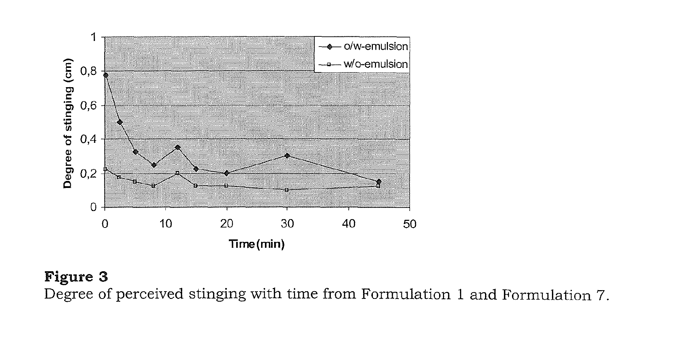

Figure 3 shows the degree of perceived stinging with time from formulation 1 and from formulation 7. The degree of stinging was marked on a 9 cm visual analogue scale. Median values, N=28. Significant differences between maximum degree of stinging (p=0.007) and area under curve (AUC) (p=0.002).

**Detailed description of the invention**

*Definitions*

For the purpose of the present invention the term topical includes topical application to the human skin in general, and particularly the skin on the face, neck, chest, back, arms, armpits, hands, feet and scalp, and also to mucous membranes, such as of the nose and genitals, especially female genitals.

As used herein the term stinging is intended to collectively refer to adverse subjective sensations, such as smarting, burning and stinging sensations, which may be experienced by the individual immediately after application of a topical formulation, especially on the facial skin, which is more sensitive to external stressors than other body regions, possibly due to a less efficient barrier with smaller number of stratum corneum cell layers and the presence of large follicular pores.

For the purpose of the present invention stinging agents include alpha hydroxy acids (AHAs), preservatives (e.g. benzoic acid and sorbic acid), humectants (e.g. lactic acid, propylene glycol, urea and PCA), and also topical pharmaceutically active agents, such as retinoic acid and benzoyl peroxide, which are known to induce inconvenient sensory reactions, and any other agents known to induce stinging upon topical application of a formulation containing same, and mixtures thereof. In the present formulation the stinging agent is included in an concentration producing a stinging sensation upon application thereof when included in conventional topical formulation. Such concentration is typically 0.5%. Accordingly, the present formulation preferably includes at least a total concentration of 0.5% by weight, more preferably at least 1% by weight, of one or more a stinging agents.

As used herein the term topical formulation relates to both cosmetic and pharmaceutical formulations for topical application.

The terms oil and emollient will be used interchangeably herein.

*Stinging and type of emulsion*

The present inventors note that it could be inferred from Sah et al that the w/o formulation disclosed could be expected to produce a reduced degree of stinging immediately after application thereof to the skin, as compared to the corresponding o/w and w/o/w emulsions. However, on the other hand, based on the data presented in Figure 9, showing amounts of lactic acid in the different layers of porcine skin, when applying the commonly acknowledged theory on skin penetration (stating a correlation between the amount in the stratum corneum and the penetration rate, i.e. amount in the receptor), the above conclusion seems questionable, since the amount of lactic acid absorbed into the receptor was higher after application of w/o than o/w, and the amount in dermis was higher than after application of w/o/w. Hence, the data presented in Fig. 9 may be indicative of influences from other unknown factors in the experimental set up.

In order to establish whether the type of formulation (i.e. w/o, o/w and w/o/w) influences the stinging, the present inventors performed the below testing, wherein different types of emulsions containing lactic acid were tested on test subjects, and the perceived degree of stinging reported.

Materials and methods

**Study design and test subjects**

The study was controlled, double-blind, randomized and bilateral on healthy volunteers. The subjects considered themselves healthy and no signs of skin disorders could be noted upon visual inspection of their skin.

**Test materials**

Four different formulations, listed in Table 1 below, were analysed. The first formulation (1) was used as a reference and was compared to the other three formulations in order to distinguish the effect from lactic acid to the effect from pH (4), lipid concentration (5), and the emulsion type, w/o (7).

**Table 1**

| Formulation | Emulsion type | pH | Lactic acid concentration, % by weight | Lipid concentration, % by weight |
|---|---|---|---|---|
| 1 | o/w | 3 | 15% | 10% |
| 4 | o/w | 3 | 0% | 10% |
| 5 | o/w | 3 | 15% | 50% |
| 7 | w/o | 3 | 15% | 10% |

The formulations in Table 1 were composed of paraffin oil, PEG-20 glyceryl stearate, xanthan gum, lactic acid and PEG-30 Dipolyhydroxystearate in the amounts given in Table 2. pH was adjusted by the addition of hydrochloric acid or sodium hydroxide.

**Table 2.** Composition of the studied formulations.

| Formulation | Paraffin oil | PEG-20 Glyceryl stearate | PEG-30 Dihydroxystearate | Xanthan Gum | Lactic Acid | Aqua ad |
|---|---|---|---|---|---|---|
| 1 | 10% | 5% | - | 0.3% | 15.0% | 100% |
| 4 | 10% | 5% | - | 0.3% | | 100% |
| 5 | 50% | 5% | - | 0.3% | 15.0% | 100% |
| 7 | 10% | - | 5% | 0.3% | 15.0% | 100% |
| Percentages are by weight | | | | | | |

**Sensory perception of the stinging potential**

The volunteers assessed the degree of stinging of one test formulation and one reference formulation simultaneously at each occasion. Before the study, the subjects cleaned their faces with mild soap and water. The cheeks were also gently cleaned twice on each side with paper napkins soaked in ethanol some minutes before application of the test formulations. 20 cm$^2$ was marked on each cheek/nasobial fold, thereafter 2 $\mu$l formulation/cm$^2$ was gently spread over the area.

The subjects were then instructed to mark their experienced degree of stinging on a ~9 cm visual analogue scale (VAS), where 0 cm represented no stinging and ~9 cm represented unbearable stinging, after 10 s, 2.5 min, 5 min, 8 min, 12 min, 15 min, 20 min, 30 min and 45 min. They also marked their opinion of the severity on a category scale (0 = no stinging; 1 = weak stinging; 2= moderate stinging and 3 = severe stinging).

**Calculations and statistics**

The degree of stinging expressed on a visual analogue scale (VAS) was plotted against time. The maximum value of the response was noted. The area under the curve (AUC) for each subject was calculated in order to investigate the total stinging response for the different formulations over time. AUC was calculated according to the following formula:

$$AUC = \sum_{i=1}^{n} \frac{\left(l(t_n) + l(t_{n-1})\right) \cdot \left(t_n - t_{n-1}\right)}{2}$$

Where $l(t_n)$ denotes the length marked in centimetres at a certain time $t_n$ after the application of the formulation.

The Wilcoxon rank sum test together with corresponding two-sided 95% confidence interval and associated Hodges-Lehmann estimator (point estimate) were calculated as a test and description of treatment differences. The Hodges-Lehmann estimator (point estimate) is calculated as the median of all possible pairs of data from the two treatment groups and is an alternative to the difference in mean or median describing the difference between two distributions.

**Results**

The majority of the subjects did not show any visual signs of erythema even though they perceived stinging in their faces. Some individuals showed barely perceptible erythema, and in a few cases more obvious erythema was noted. The perception of stinging reached a peak value during the first minutes, as can clearly be seen from the o/w-emulsions in Figures 1-3.

Removal of lactic acid from the formulation and adjustment of the formulation to pH 3 with hydrochloric acid (Formulation 4) resulted in a significantly lower degree of stinging measured as AUC (n=20; p=0.022). Also measurement of the difference in maximum values gave significant results (n=20; p=0.003).

The difference in stinging between the reference Formulation 1 containing 10% of fat and Formulation 5 containing 50% fat was not significant (n=19; p= 0.251) when AUC was assessed with the VAS-scale (Table 3, Figure 2). However, it showed tendencies towards a lesser degree of stinging reported for the formulation with a higher concentration of fat and so did the difference in maximum values reported on the VAS-scale (n=19; p=0.077), Table 3.

The difference reported between water-in-oil (w/o) emulsion and reference oil-in-water (o/w) emulsion was prominent, as can be clearly seen from Figure 3. The difference in AUC reported on the VAS-scale was significant (n=28; p=0.006) and even more pronounced regarding maximum value of the stinging capacity (n=28; p=0.002).

**Table 3.** Point estimate of the treatment difference between the study formulations, with the corresponding 95% confidence interval given within parenthesis.

| Stinging parameter | pH 3 from 15% lactic acid vs. HCl (n=20) (Formulation 1 vs. 4) | 10% vs. 50% (n=19) (Formulation 1 vs. 5) | o/w vs. w/o (n=28) (Formulation 1 vs. 7) |
|---|---|---|---|
| Area under curve (AUC), p-value | 1.85 (0.28; 9.25) 0.022 | 0.90 (-2.51; 23.1) 0.251 | 3.34 (1.8; 20) 0.006 |
| Max, p-value | 0.40 (0.175; 0.825) 0.003 | 0.550 (-0.05; 1.25) 0.077 | 0.575 (0.25; 1.48) 0.002 |

**Discussion**

Assessment of the stinging capacity of topically used substances usually includes application of the test agent to the nasolabial fold in 5-10 individuals sensitive to 5% lactic acid. In the present study we included normal healthy individuals and made the tests at ambient conditions. To increase the possibility to detect stinging, we used 15% lactic acid in the formulations and included not less than 19 individuals. Furthermore, the degree of stinging was marked on a continuous line, which increases the sensitivity of the measurement compared to the use of category scales.

Removal of lactic acid and acidification of the formulation by addition of hydrochloric acid to the same pH as in the lactic acid emulsion (pH 3) almost eliminated the stinging potential of the o/w-emulsion (Figure 1). This finding emphasizes the inherent stinging potential from lactic acid and does also show that pH 3 from a non-buffered solution may be tolerated by the skin, probably due to the inherent buffering capacity of skin.

Sweating is reported to enhance the possibility to detect stinging. This change in stinging threshold might be due to increased hydration, open pores or changed solubility of the stinging substances due to the water layer on the skin surface. In agreement with previous findings, we noted stinging immediately after application of the emulsions, which lasted for about 10 minutes. During this period, water and other volatile substances will evaporate from the skin surface, and a substantial increase in the concentration of lactic acid and other non-volatile substances will occur in the applied layer on the surface. Furthermore, a minor part of the emulsion water and some lactic acid are also absorbed into the skin and give rise to skin reactions.

Increasing the fat content in the o/w-emulsion tended to reduce the degree of stinging, although the difference was not significant. Changing the amount of fat in the emulsion from 10 to 50% will also increase the concentration of lactic acid in the water phase. The actual change in the concentration of lactic acid will depend on the solubility of lactic acid in the fat phase and the resultant partition between the two phases. Increased concentration of lactic acid in the water phase should in theory favour its stinging potential, due to larger amount of lactic acid absorbed into the skin. However, this may be counteracted by the applied fat layer and the reduced amount of water in the formulation. With the same rate of application (2 $\mu$l/cm$^2$), less water will be applied on the surface and this water may evaporate more rapidly from the formulation than from the emulsion with lower fat content. Previous findings indicate that water on the surface favours absorption of lactic acid, since sweating enhances the degree of stinging. Hence, the effect on stinging is likely to be a multifactor event and the resultant effect may not be easy to predict.

*Follicular transport*

Usually skin appendages do not contribute to any significant extent to the absorption of substances. However, the present inventors have found skin appendages, such as follicles, to be associated with stinging. According to the present inventors, the rapid onset of stinging suggests penetration of lactic acid via skin appendages and not via the intercellular route. The appendages are believed to facilitate penetration of stinging agents, such as lactic acid, through the skin, and thereby promoting a stinging sensation perceived by the user immediately upon application by allowing for an easier diffusion pathway in parallel to the normal route through the stratum corneum *per se.* For the most part their effect on skin permeability is minimized owing to their relatively small total fractional area (ca. 0.1%). However, as an early stage event the appendages may serve for rapid passage, where the intercellular pathway is dominant for the majority of substances after reaching steady-state.

By preventing penetration of stinging agents via this pathway, stinging can be reduced. According to the present invention this is achieved by using in a w/o emulsion microparticles effective for follicular closure, thereby blocking or substantially inhibiting follicular transport.

The size of the particles should generally be selected so as to be effective for follicular closure. For this purpose a suitable size is considered to be 1-10 $\mu$m, and more preferably 5-6 $\mu$m, such as for example about 5 $\mu$m. The particles can be formed from primary particles of a smaller size. Accordingly, primary particles of a smaller size, which will agglomerate to form lager complexes within the above range in the formulation, can also be used when preparing the formulation. The primary particles can be of a size of about 0.01 $\mu$m or more, such as 0.01-1 $\mu$m. Any conventionally used microparticles in cosmetics, such as for example metal oxides such as calcium oxide, titanium dioxide and zinc oxide, or talc, or polymeric materials based on silicon, acrylates or polyethylene can be used according to the present invention. The microparticles are included in an amount within the range of 0.5-20%, more preferably 1-10%, and most preferably 2-5%.

The microparticles will also impart a more powdery and dry feel to the formulation, and thus improved cosmetic properties. Thereby, mineral oils, such as paraffin oil, could be used as emollient, since the dry feel would counteract or reduce the tackiness associated with the inclusion of mineral oils.

The presence of microparticles, such as within the range of about 1-10 $\mu$m, in the topical formulation, will generally also give rise to a whiteness of the formulation, which will typically make the formulation visible upon application to e.g. the skin.

*Emollients*

The emollients of the present invention are not critical and can be selected from any conventionally used emollients, e.g. mineral oils, such as for example paraffin oil, alkyl or alkenyl esters, such as isopropyl myristate and ethylhexyl stearate or similar, and preferably blends thereof, silicone oils, both volatile and non-volatile (cyclomethicone and dimethicone), triglycerides such as caprylic/capric triglyceride, and other vegetable oils.

From an environmental standpoint the use of oils based on non-renewable raw materials, such as paraffin oil, should be minimized, and consequently, the use vegetable oils is preferred.

The oil used in the present invention can be used in conventionally used amounts.

Creams (o/w-emulsions) are the most popular delivery forms for topical preparations. The inner phase of emulsions conventionally constitutes about 15-25% of the formulation. Any active ingredients can be dissolved in the oil-phase or in the water-phase, depending on the solubility of the actives.

The high consumer acceptability of o/w-creams is due to the possibility to easily spread the fatty material and actives in a thin layer on the surface without inducing a greasy surface. Typically, approximately 1-2 mg of cream is applied per sq cm. Since the outer phase of the emulsions usually is water and constitutes 75-85% of the formulation, only about 0.2-0.5 mg/sq cm of non-volatile material from the inner phase will be deposited on the skin. Inverse emulsions with water in the inner phase and fatty material in the outer *(i.e.* w/o-emulsions) are traditionally considered greasy due to the much higher level of fatty material in the formulation, i.e. in the outer phase.

*The emulsifier of the inventive formulation*

Any conventionally used emulsifier in the field of cosmetics and pharmaceuticals can be used according to the present invention. In order to secure a better stability of the emulsion upon storage the emulsifier should exhibit an HLB of <10.

The water content of a w/o emulsion can be increased by enlargement of the inner phase, but this enlargement induces deformations of the droplets with obvious risks for phase separation. However, with the advent of recently developed w/o-emulsifiers, the droplets in the inner phase can be deformed without inducing instability of the emulsion. The use of such emulsifiers makes it possible to use similar levels of fat in w/o-emulsions as in o/w-emulsions, thus further improving the cosmetic properties of the inventive emulsions.

Preferred emulsifiers of the above type used in the invention for further improving the cosmetic properties are hydrofobically and hydrofilically (ethoxylated) modified polydimethylsiloxane backbones, especially cetyl PEG/PPG 10/1 dimethicone and bis-PEG/PPG-14/14 dimethicone. Another useful ethoxylated emulsifier is PEG-30 dipolyhydroxystearate. Derivatives of nonethoxylated components, especially polyglyceryl derivatives, such as polyglyceryl-3 polyrici-noleate and polyglyceryl-4 isostearate, polyglyceryl-2 dipolyhydroxystearate and sorbitan esters, such as for example sorbitan oleate, are also suitable as emulsifiers and/or coemulsifiers. The emulsifiers can also be used in combinations to obtain synergistic effects on the demanded properties, such as increased storage stability, increased spreadability on the skin as well as cosmetic acceptance in general.

The above preferred emulsifiers have an optimised mixture of hydrophilic and hydrophobic parts to form stable w/o emulsions with a relatively low oil content, which allows for inclusion of a large inner-phase of water without inducing instability and a too short shelf-life to the products.

A concentration of the above emulsifiers of 0.5-6 %, especially 1-4 %, such as for example 4 %, in the inventive formulation, will provide a stable emulsion with a desired shelf-life, and increased robustness to high shearing forces. Furthermore, the reduced interfacial tensions facilitate uniform coverage of surface defects, and piling of emollient material in the surface orifices. This allows the content of emollients in the inventive emulsion to be kept low, preferably 15-25%.

Nonionic emulsifiers are the generally preferred stabilizers for the inventive emulsions due to their mildness. However, ethoxylated emulsifiers may be susceptible to oxidation, inducing formation of peroxides and aldehydes, such as formaldehyde, which is known to be hazardous to the human body. From this viewpoint, however, ethoxylated emulsifiers (such as PEGs) should preferably be avoided in the inventive formulations.

During the development work it was found that especially attractive cosmetic properties can be achieved with the above new type of emulsifiers when a balanced combination of the above emulsifiers and the emollients are chosen. For the invention the amount of liquid oils (at room temperature) are important as well as the spreadability of the oils on the skin. The more liquid the oil and the higher the spreadability, the more easily they will cover the orifices and fissures of the skin. The preferred amounts of liquid emollients are 5-35 %, more suitable 10-30 and especially 15-25 % based on the overall formulation.

The content of waxes (not liquid at room temperature) may typically be within the range of 1-2 %.

*Consistency enhancers and thickeners*

Advantageously, the above listed emulsifiers have also been found to reduce the importance of thickeners and emulsion stabilisers in the formulation, allowing for lower amounts of stabilisers, consistency enhancers and thickeners to be used (such as within the range of 0-10 %, more preferably 0-4%, such as 1-4%, and most preferably 0-3%, such as e.g. 2-3%), while stable emulsions are still obtained. The reduced amount makes the liquid oils in the formulation more available for spreading on the skin, which oils will therefore more easily cover the surface defects and orifices in the skin.

Suitable consistency enhancers and thickeners can be chosen from all types of cosmetically acceptable conventionally used thickeners and consistency enhancers. These include polymers such as polybutene and silica, wax esters such as beeswax and myristyl myristate, fatty alcohols such as cetyl and stearyl alcohols or blends thereof, hydrogenated vegetable oils, such as for example hydrogenated castor oil and hydrogenated coco-glycerides, and hydrofobically modified or crosslinked polydimethylsiloxanes such as cetyl dimethicone and dimethicone crosspolymer.

*Osmotically active agents*

In order to further avoid separation of the w/o emulsions different salts or osmotically active ingredients are preferably added to the water phase, preferably sodium chloride and/or magnesium sulphate. Useful concentrations are 0.01-5%, and preferably 0.1-1%.

*Droplet size*

The final consistency of the emulsion is also dependent of the droplet size and distribution. By adjusting the homogenisation time and speed the viscosity can be adjusted as known in the art so that a heavy cream or a thin lotion is formed, as desired dependent on the intended application. A small and uniform droplet size of 20-30 $\mu$m is especially preferred with regard to the stability of the emulsion upon storage.

*Cosmetic and pharmaceutical use*

As will be apparent to the skilled person reading the present disclosure, the present formulation having reduced stinging potential can advantageously be formulated for use as a pharmaceutical. Accordingly, the above described embodiments can be formulated either for use as a cosmetic or as a pharmaceutical. In this regard, it is also believed that penetration of stinging agents through the skin via fissures will also be inhibited by the inventive formulation.

Facial skin is more sensitive to external stressors than other body regions possibly due to a less efficient barrier with smaller number of stratum corneum cell layers and the presence of large follicular pores. Sunburned skin, atopics and other patients with eczema are also at a higher risk for further skin reactions, due to the already excited skin and/or increased permeability. For example, when atopics are asked to judge the degree of adverse skin reactions to urea-containing moisturizers 20-40% report stinging sensations (a sharp, local, superficial effect similar to the reaction noted to acidic solutions). Combinations with corticosteroids do not eliminate stinging and 12-30% of patients using hydrocortisone creams with 4-20% urea on eczema also reports stinging.

For patients with skin diseases, the process of treating the skin with topical medications adds to the burden of having the disease. Not only disagreeable cosmetic features, such as greasy compositions and bad odour from ingredients, but also sensory skin reactions can make the treatment inconvenient and lower the compliance. Moreover, long-term treatment of atopic children with moisturizers may also have an impact on emotional development depending on the quality of the interaction between the parent and child. Creaming may feel soothing and comforting, punitive and intrusive, or functional and neutral.

Most of the efforts to increase the compliance hitherto have been focusing on the cosmetic properties of the formulation. For example, ointments are being replaced by creams and other non-greasy and more easily applied formulations. Moreover, ingredients that can cause adverse reactions are avoided as far as possible. Less emphasis has been put on the possibility to reduce absorption of the unwanted substances from the formulation, although it is well-known that the bioavailability of added ingredients is influenced by ingredients in the formulation. More often penetration enhancers are used to increase skin absorption. The enhancers are targeted to modify the composition or organization of the intercellular lipid bilayers, which is the rate-limiting barrier to penetration of substances through the stratum corneum.

According to the present invention, the added ingredient, i.e. the microparticles, required to reduce the stinging sensation is essentially inert. This is believed to be particularly advantageous in pharmaceutical applications.

The formulation of the present invention can be used for preparing cosmetics such as creams, ointments, lotions, and also pharmaceuticals based on the inventive emulsion, such as creams, ointments and lotions. The latter can be intended for topical application to the skin, or for application to mucous membranes such as of the nose or genitals, and formulated as e.g. a nasal spray.

*Lactic acid*

While lactic acid is a particularly stinging ingredient, as compared to other more moderately stinging ingredients such as for example urea, it is also known for its beneficial dermatological effects, such as its exfoliant properties. The effects can be seen as purely cosmetic (e.g. dryness), or belong to the medicinal area and be used for prevention or treatment of hyperkeratotic skin (e.g. patient with ichthyosis, lichenified eczema or psoriasis). Furthermore, the exfoliant properties can also be used for chemical peeling of uneven, but otherwise normal, skin. Similar dermatological effects have also been attributed to other alpha hydroxy acids (AHAs), such as glycolic acid, malic acid, citric acid, and tartaric acid. The AHAs are known in the art to be stinging ingredients. In prior art formulations, lactic acid is known to produce a stinging action at concentrations of about 0.5% by weight of the formulation.

In a preferred embodiment the inventive formulation contains one or more AHAs in an amount of 1 to 30% by weight of the formulation.

Accordingly, in a more preferred embodiment the present formulation contains lactic acid as an AHA. Lactic acid is preferably contained in an amount ranging from about 1 to 30%, more preferably 2-15%, and especially 5 to 10% by weight of the inventive formulation.

*pH value of the formulation*

While not wishing to be bound by any theories, the present invention is believed to be particularly useful for reducing the stinging action of AHAs, and particularly lactic acid. Also, from the point of stinging potential, it has been found that the pH of the resulting formulation, usually in the range of the pKa of the AHA used, does not require to be further adjusted, such as by partial neutralization.

The pH value of the inventive formulation may be in the general range of 2.0 to 10. The inventive compositions are particularly useful when they are at an acidic pH, preferably 2.5-6.

*Stinging agents*

As already stated above, it is believed that the stinging potential of the stinging agent is decreased by reducing the immediate absorption of the agent (via the follicular pathway. This penetration pathway is however of negligible importance when the desired beneficial effect of the acids is exerted). Only excipients contributing to excellent cosmetic properties and with demonstrated safety are used to manufacture the formulations in the present invention.

For patients with skin diseases, the process of treating the skin with topical medications adds to the burden of having the disease. Disagreeable cosmetic features and inconvenient skin reactions can make the treatment troublesome and lower the compliance. By using the formulation of the present invention in topical pharmaceutical formulations, the tolerability of the formulations will be markedly enhanced.

The formulation will allow for higher concentrations of such any stinging active substance to be used, while an improved tolerability of the formulation is achieved.

Accordingly, in addition to the AHAs mentioned above, the stinging ingredient can be selected from any other substances known to cause subjective sensations, such as benzoic acid and sorbic acid, conventionally used as preservatives, in an amount of from 0.3 to 1.5 % by weight of the formulation, or propylene glycol, urea and PCA, conventionally used as humectants, in an amount of from 2 to 10 % by weight of the formulation. Furthermore, topical pharmaceutically active agents, such as retinoic acid and benzoyl peroxide, which are known to induce inconvenient sensory reactions, may be included in pharmaceutically effective concentrations, such as an amount ranging from 0.1 to 10 % by weight of the formulation.

*Optional additives*

Additionally, the formulation of the present invention may contain any conventionally used additives such as preservatives, emulsifiers, skin-feel agents, humectants, oils, lipids, colours, particles, pH-adjusters, complexing agents, thickeners, surfactants, vitamins or other actives.

In one embodiment the formulation includes a conventionally used sunscreen, *i.e.* a substance used to block ultraviolet radiation.

*Preparation*

The formulations of the invention can be prepared using any conventionally known methods for preparing w/o-emulsions. In the methods water and oil soluble ingredients are most often blended in separate vessels (this might require heating to about 60-90°C if the ingredients are solid at room temperature or difficult to solubilise). The water is then slowly transferred under moderate stirring to the oil/wax vessel (at 10-2000 rpm depending on equipment). After transfer of water phase the blend is homogenised (at 2000-20000 rpm). The viscosity, stability and properties of the product are dependent on the shear force used, as will be realised by the skilled person. However, it will be appreciated by the person skilled in the art that any method which will produce a w/o emulsion in which the stinging ingredient is contained in the water phase can be used to prepare the inventive formulation.

The invention will now be described with reference to the following examples, wherein A denotes the aqueous phase and B denotes the oily phase, which are provided only for illustrating purposes, and should not be construed as limiting the scope of the invention thereto.

The particles can be added to the aqueous or oily phase, depending on the hydrophobicity thereof.

In the Examples the following general procedure was used to prepare the formulations. The constituents of the phases A and B, respectively, are mixed, and each phase is heated separately to 75°C. Phase A is thereafter added to phase B slowly under moderate stirring. The emulsion is homogenized at low speed (6500 rpm) for 2 min. Thereafter the emulsion thus formed is allowed to cool to room temperature under stirring.

## Examples

**Example 1** (hydrophobic $TiO_2$ particles)

| Phase | Constituent | % w/w | Remark |
|---|---|---|---|
| A | Water | 68,2 | |
| A | Glycolic Acid | 5 | stinging agent |
| A | Sodium Chloride | 0,8 | osmotically active agent |
| B | Polyglyceryl-4 Isostearate | 1 | emulsifier |
| B | Cetyl PEG/PPG-10/1 Dimethicone | 3 | emulsifier |
| B | Hydrogenated coco-glycerides | 1 | consistency enhancer/thickener |
| B | Hydrogenated castor oil | 1 | consistency enhancer/thickener |
| B | Dimethicone | 3 | emollient |
| B | Paraffinum Perliquidum PhEur | 15 | emollient |
| B | Tioveil 50 FCM | 2 | Uniqema; particle size 40 nm (20-200 nm) |

**Example 2** (hydrophobic $TiO_2$ particles)

| Phase | Constituent | % w/w | Remark |
|---|---|---|---|
| A | Water | 68,2 | |
| A | Sodium Chloride | 0,8 | osmotically active agent |
| A | Citric Acid | 5 | stinging agent |
| B | Polyglyceryl-4 Isostearate | 1 | emulsifier |
| B | Cetyl PEG/PPG-10/1 Dimethicone | 3 | emulsifier |
| B | Hydrogenated castor oil | 1 | consistency enhancer/thickener |
| B | Beeswax | 1 | consistency enhancer/thickener |
| B | Dimethicone | 3 | emollient |
| B | Paraffinum Perliquidum PhEur | 15 | emollient |
| B | Solaveil Clarus | 2 | Uniqema; particle size 30 nm (20-100 nm) |

**Example 3** (acrylate particles)

| Phase | Constituent | % w/w | Remark |
|---|---|---|---|
| A | Water | 65,2 | |

(continued)

**Example 3** (acrylate particles)

| Phase | Constituent | % w/w | Remark |
|---|---|---|---|
| A | Sodium Chloride | 0,8 | osmotically active agent |
| A | Malic Acid | 5 | stinging agent |
| B | Polyglyceryl-4 Isostearate Cetyl PEG/PPG-10/1 Dimethi- | 1 | emulsifier |
| B | cone | 3 | emulsifier |
| B | Hydrogenated coco-glycerides | 1 | consistency enhancer/thickener |
| B | Beeswax | 1 | consistency enhancer/thickener |
| B | Dimethicone | 3 | emollient |
| B | Paraffinum Perliquidum PhEur | 15 | emollient |
| B | SP-500 | 5 | Kobo Products; particle size <10 $\mu$m |

**Example 4** (ZnO particles)

| Phase | Constituent | % w/w | Remark |
|---|---|---|---|
| A | Water | 65,2 | |
| A | Sodium Chloride | 0,8 | osmotically active agent |
| A | Lactic Acid | 5 | stinging agent |
| B | Polyglyceryl-4 Isostearate Cetyl PEG/PPG-10/1 Dimethi- | 1 | emulsifier |
| B | cone | 3 | emulsifier |
| B | Hydrogenated castor oil | 1 | consistency enhancer/thickener |
| B | Beeswax | 1 | consistency enhancer/thickener |
| B | Dimethicone | 3 | emollient |
| B | Paraffinum Perliquidum PhEur | 15 | emollient |
| B | Spectraveil Fin | 5 | Uniqema; particle size 400-1000 nm |

**Example 5** (hydrophilic $TiO_2$ particles)

| Phase | Constituent | % w/w | Remark |
|---|---|---|---|
| A | Water | 68,2 | |
| A | Sodium Chloride | 0,8 | osmotically active agent |
| A | Lactic Acid | 5 | stinging agent Uniqema; particle size 40 nm (20- |
| A | Tioveil 50 AQ-N | 2 | 200 nm) |
| B | Polyglyceryl-4 Isostearate | 1 | emulsifier |
| B | Cetyl PEG/PPG-10/1 Dimethicone | 3 | emulsifier |
| B | Hydrogenated castor oil | 1 | consistency enhancer/thickener |
| B | Beeswax | 1 | consistency enhancer/thickener |
| B | Dimethicone | 3 | emollient |
| B | Paraffinum Perliquidum PhEur | 15 | emollient |

**Example 6** (talc particles)

| Phase | Raw Material | % w/w | |
|---|---|---|---|
| A | Water | 68,2 | |
| A | Sodium Chloride | 0,8 | osmotically active agent |
| A | Lactic Acid | 5 | stinging agent |
| A | Talc | 2 | particle size <40 $\mu$m |

(continued)

**Example 6** (talc particles)

| Phase | Raw Material | % w/w | |
|---|---|---|---|
| B | Polyglyceryl-4 Isostearate Cetyl PEG/PPG-10/1 Dimethi- | 1 | emulsifier |
| B | cone | 3 | emulsifier |
| B | Hydrogenated coco-glycerides | 1 | consistency enhancer/thickener |
| B | Beeswax | 1 | consistency enhancer/thickener |
| B | Dimethicone | 3 | emollient |
| B | Paraffinum Perliquidum PhEur | 15 | emollient |

* 99% of the particles having a size <40 $\mu$m, with a predominant fraction within the interval of 1-10 $\mu$m

The formulations were tested and their spreading behaviour on the stratum corneum was shown to be non-tacky and with agreeable cosmetic properties. By using the particles of the invention better cosmetic properties of the formulation are also obtained, especially a better skin feel, as compared to for example the formulation used by Sah et al

For the compositions of Examples 4, 5 and 6 containing lactic acid, it was noted that the inclusion of microparticles further reduced the degree of stinging from lactic acid in the formulation, as compared to a similar formulation of the prior art containing lactic acid, i.e. without microparticles.

**Claims**

1.   Formulation for topical application having reduced stinging potential and improved feel comprising a w/o emulsion of an emollient-containing oily phase and an aqueous phase, containing a stinging agent, and an emulsifier, **characterised in** containing 0.5-20% by weight of microparticles of a size effective for follicular closure, preferably within a range of 1-10 $\mu$m.

2.   The formulation of claim 1, including a total concentration of at least 0.5% by weight, more preferably at least 1% by weight, of one or more stinging agents.

3.   The formulation of claim 1 or 2, wherein the stinging agent is selected from AHAs, benzoic acid, sorbic acid, propylene glycol, urea, pyrrolidone carboxylic acid (PCA).

4.   The formulation of any of the previous claims, wherein the emulsifier is selected from the group consisting of hydrofobically and hydrofilically modified polydimethylsiloxane backbones, PEG-30 dipolyhydroxystearate, and polyglyceryl derivatives.

5.   The formulation of any of the previous claims, wherein the emulsifier is nonethoxylated.

6.   The formulation of any of the previous claims, including as a stinging agent one or more alpha hydroxy acids (AHAs) in a total amount of 1-30% by weight of the formulation, more prefarably 2-15%, and most preferably 5-10% by weight of the formulation.

7.   The formulation of claim 5 or 6, wherein the AHA is lactic acid.

8.   The formulation of any of claims 5-7, the pH of which is essentially that of the pKa of the one or more AHAs.

9.   A topical formulation of any of the preceding claims containing a pharmaceutically active ingredient for topical administration, for use as a pharmaceutical.

10.   A topical formulation of claim 1, including as stinging agent at least 0.5% by weight, more preferably at least 1 % by weight, of one or more pharmaceutically active ingredients for topical administration, for use as a pharmaceutical.

11.   A topical formulation of any of the claims 1-8, for use as a cosmetic.

**12.** Use of microparticles effective for follicular closure in a formulation for topical application comprising a w/o emulsion for reducing the stinging potential of a stinging agent included in the formulation.

**13.** Use of a w/o emulsion containing microparticles effective for follicular closure, for preparing a topical formulation containing a stinging ingredient having reduced stinging.

**14.** The use of claim 12 or 13, wherein the formulation is a cosmetic.

**15.** The use of claim 12 or 13, wherein the formulation is a pharmaceutical.

**Figure 1**
Degree of perceived stinging with time from Formulations 1 and 4, respectively.

**Figure 2**
Degree of perceived stinging with time from Formulations 1 and 5, respectively.

**Figure 3**
Degree of perceived stinging with time from Formulation 1 and Formulation 7.

**European Patent Office**

**EUROPEAN SEARCH REPORT**

Application Number

EP 06 12 6376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 02 167212 A (SHISEIDO) 27 June 1990 (1990-06-27) * abstract * ----- | 1-3,5,6, 8-14 | INV. A61K8/06 A61K8/25 A61K8/27 |
| X | EP 0 856 305 A2 (L'OREAL) 5 August 1998 (1998-08-05) * examples * ----- | 1-14 | A61K8/29 A61K8/365 A61K8/81 A61Q19/00 |
| Y | WO 98/56345 A (PROCTER & GAMBLE) 17 December 1998 (1998-12-17) * examples * ----- | 1-15 | |
| Y | EP 0 691 126 A (BEIERSDORF) 10 January 1996 (1996-01-10) * examples 1-5 * ----- | 1-15 | |
| Y | EP 0 456 459 A2 (UNILEVER) 13 November 1991 (1991-11-13) * page 5, line 40 - page 6, line 40 * * examples * ----- | 1-15 | |
| Y,D | WO 01/13877 A (UNILEVER) 1 March 2001 (2001-03-01) * page 42, line 1 - line 21 * ----- | 1-15 | **TECHNICAL FIELDS SEARCHED (IPC)** A61K |
| A | DE 198 05 827 A1 (BEIERSDORF) 19 August 1999 (1999-08-19) * page 3, line 31 - line 41 * * page 6, line 3; claims * ----- | 1-15 | |
| Y | EP 0 780 112 A (BEIERSDORF) 25 June 1997 (1997-06-25) * page 12, line 18 - line 22 * ----- | 1-15 | |
| Y | US 2006/110414 A1 (SHISEIDO) 25 May 2006 (2006-05-25) * page 5, paragraph 85 * ----- | 1-15 | |

The present search report has been drawn up for all claims

| Place of search | Date of completion of the search | Examiner |
|---|---|---|
| Munich | 3 July 2007 | Irwin, Lucy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 12 6376

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2007

| Patent document cited in search report | | Publication date | Patent family member(s) | | Publication date |
|---|---|---|---|---|---|
| JP 2167212 | A | 27-06-1990 | JP | 2919495 B2 | 12-07-1999 |
| EP 0856305 | A2 | 05-08-1998 | CA | 2225587 A1 | 03-08-1998 |
| | | | DE | 69828093 D1 | 20-01-2005 |
| | | | DE | 69828093 T2 | 15-12-2005 |
| | | | ES | 2234069 T3 | 16-06-2005 |
| | | | JP | 10306014 A | 17-11-1998 |
| | | | US | 5843417 A | 01-12-1998 |
| WO 9856345 | A | 17-12-1998 | AU | 742341 B2 | 20-12-2001 |
| | | | AU | 8257698 A | 30-12-1998 |
| | | | CA | 2293330 A1 | 17-12-1998 |
| | | | CN | 1265024 A | 30-08-2000 |
| | | | EP | 0996417 A1 | 03-05-2000 |
| | | | JP | 2001515514 T | 18-09-2001 |
| EP 0691126 | A | 10-01-1996 | DE | 4423450 A1 | 11-01-1996 |
| | | | JP | 8026972 A | 30-01-1996 |
| EP 0456459 | A2 | 13-11-1991 | AU | 636483 B2 | 29-04-1993 |
| | | | AU | 7640691 A | 14-11-1991 |
| | | | CA | 2041917 A1 | 11-11-1991 |
| | | | DE | 69101466 D1 | 28-04-1994 |
| | | | DE | 69101466 T2 | 14-07-1994 |
| | | | DK | 456459 T3 | 25-07-1994 |
| | | | ES | 2062683 T3 | 16-12-1994 |
| | | | GB | 2243780 A | 13-11-1991 |
| | | | IN | 172888 A1 | 25-12-1993 |
| | | | JP | 4226906 A | 17-08-1992 |
| | | | JP | 7045375 B | 17-05-1995 |
| | | | US | 5196187 A | 23-03-1993 |
| | | | ZA | 9103552 A | 27-01-1993 |
| WO 0113877 | A | 01-03-2001 | AU | 6988100 A | 19-03-2001 |
| DE 19805827 | A1 | 19-08-1999 | EP | 0937454 A2 | 25-08-1999 |
| EP 0780112 | A | 25-06-1997 | DE | 19548013 A1 | 26-06-1997 |
| | | | JP | 9175934 A | 08-07-1997 |
| | | | US | 2002077372 A1 | 20-06-2002 |
| US 2006110414 | A1 | 25-05-2006 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0113876 A
- US 6440432 B

- WO 0113877 A

**Non-patent literature cited in the description**

- An in vitro study of the effects formulation variables and product structure on percutaneous absorption of lactic acid. *J. Cosmet. Sci,* 1998, vol. 49, 257-273